# EUROPÄISCHE PATENTANMELDUNG

(11) **EP 3 960 728 A1**
(43) Veröffentlichungstag der Anmeldung: **02.03.2022**
(21) Anmeldenummer: 20192391.9
(22) Anmeldetag: 24.08.2020
(51) Int. Cl.: C07C 263/10, C07C 265/14, C01B 32/80

(54) **VERFAHREN ZUR ISOCYANAT-HERSTELLUNG MIT REZYKLIERENDER, THERMISCH OXIDATIVER VERWERTUNG**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Es wird ein Produktionsverfahren zur Herstellung von Isocyanaten beschrieben, bei welchem Produktionsrückstände, insbesondere der Destillation, durch thermische oxidative Behandlung in Verwertungsprodukte, beispielsweise Synthesegas, umgewandelt und zur Bereitstellung von Reaktanden genutzt und als solche wieder in den Produktionskreislauf eingespeist werden.

## Beschreibung

Die vorliegende Erfindung betrifft ein Verfahren, in dem Verwertungsprodukte, insbesondere Synthesegas, aus einem Produktionsrückstand der Isocyanatproduktion gewonnen und zur Bereitstellung von Reaktand für die chemische Produktion, insbesondere für die Isocyanatproduktion, genutzt werden.

Die Herstellung von Di- oder Polyisocyanaten wie Toluylendiisocyanat (TDI) erfolgt bekannterweise durch Phosgenierung, i.e. durch die Umsetzung der Reaktanden Phosgen und organisches Amin, insbesondere Toluylendiamin (TDA), wobei die Bereitstellung des dazu benötigten Phosgens üblicherweise durch eine der Phosgenierung vorgeschalteten Umsetzung der Reaktanden Chlor und Kohlenmonoxid bewirkt wird.

Das bei der Phosgenierung erhaltene rohe Isocyanat, beziehungsweise das rohe TDI, wird anschließend wie allgemein bekannt destillativ aufgereinigt.

All den bekannten Verfahren zur destillativen Reinigung des rohen TDI ist gemeinsam, dass neben dem gewünschten gereinigten TDI aus der Destillation höher siedender Komponenten erhalten werden, welche mindestens einer sachgerechten Entsorgung zuführbar gemacht werden müssen. Der Stand der Technik zur Behandlung sogenannter Destillationsrückstande der TDI-Herstellung beschreibt verschiedene Verfahren. Generelle Ziele der Rückstandsbehandlung sind die Maximierung der TDI Ausbeute, die Minimierung des Mengenanfalls an Rückstand und eine möglichst sinnvolle kostengünstige und einfache Verwertung der für den TDI-Herstellprozeß nicht mehr nutzbaren Rückstandsmenge. Die Aufarbeitung von Rückständen aus der Isocyanat Herstellung ist von steigendem wirtschaftlichem Interesse, da mit zunehmender Anlagengröße die Menge an Rückstand sowie die darin enthaltende Wertstoffmenge steigt.

Die thermisch induzierte unerwünschte Bildung von höheren Polymeren aus Diisocyanaten durch die Reaktion mit Spuren an Feuchte, Aminen sowie untereinander zu z.B. Harnstoffen, Uretdionen, Biureten, Isocyanuraten, Carbodiimden und Uretoniminen ist allgemein bekannt und vielfach in der Literatur beschrieben. Als wesentlich nachteilig an diesen unerwünschten Nebenreaktionen ist, das zum einen bei ihrer Bildung Wertstoff (TDI) verbraucht wird und es andererseits dabei zu einem unkontrollierbaren Polymeraufbau kommt welcher mit einem Viskositätsanstieg einhergeht. Dies trifft im besonderen Maße für Rückstandskonzentrationen oberhalb > 10 Gew.% in TDI zu. Ebenso führt der Polymeraufbau in vielen Fällen zu in organischen Lösungsmitteln unlöslichen Verbindungen. Somit sind solche Rückstände nur unter größerer Anstrengung und in geringen Konzentrationen in verfahrenstechnisch einfach handhabbare Lösungen zu überführen. Für solche Rückstände kommen durch ihre schlechte Handhabbarkeit nur wenige aufwendige Verfahren zum Tragen. Dies schmälert die Wirtschaftlichkeit der Aufarbeitung solcher Rückstände in erheblichem Maße.

Zur Minimierung der Isocyanatausbeuteverluste kann der Destillationsrückstand in einen gerührten und beheizten Behälter übergeführt und mit hochsiedenden Kohlenwasserstoffen, vorzugsweise Bitumen, die unter den Destillationsbedingungen inert sind, vermischt werden, um möglichst vollständig das im Rückstand noch vorhandene freie Isocyanat abzudestillieren (EP 0 548 685 A2). Der verbleibende, von Isocyanat befreite Rückstand kann als rieselfähiger Feststoff ausgetragen werden. Nachteile dieses Verfahrens sind neben dem Einsatz eines prozessfremden Stoffes (Bitumen) Ausbeuteverluste durch Polymerisation des Isocyanats, da der Prozess hohe Verweilzeiten bei hoher Temperatur beinhaltet.

Ein weiteres Verfahren zur Isocyanat-Rückstandsabtrennung ist gekennzeichnet durch den Einsatz von Knetertrocknern (EP 0 626 368 A1). In diesem Verfahren werden die oben beschriebenen beheizten und gerührten Behälter durch Knetertrockner ersetzt. Durch Einsatz von zum Beispiel Bitumen wird wie im oben genannten Beispiel der verbleibende Rückstand als rieselfähiger Feststoff erhalten der als Brennstoff zum Beispiel in Zementwerken eingesetzt werden kann.

EP 2 540 702 A2 beschreibt ein Verfahren zur Gewinnung von Toluylendiisocyanat aus flüssigen Rückständen der Toluylendiisocyanatherstellung, bei dem zunächst in einem beheizten Behälter mit Rührorgan ein feinkörniger Feststoff vorgelegt wird, so dass durch den Rührvorgang ein mechanisch induziertes Wirbelbett erzeugt wird. Das Wirbelbett wird über die Verdampfungstemperatur von Toluylendiisocyanat erhitzt, so dass beim Dosieren von flüssigen TDI-haltigen Rückständen in das Wirbelbett sich Toluylendiisocyanat aus dem Rückstand der Toluylendiisocyanatherstellung abtrennen lässt.

EP 0 699 659 A2 beschreibt ein Verfahren und eine Vorrichtung zur Abtrennung eines festen Rückstandes aus einer Lösung des Rückstandes in verdampfbaren Wertstoffen und/oder Lösungsmitteln unter Zusatz von bis zu 20 Gew.-% hochsiedenden Kohlenwasserstoffen, die unter Verdampfungsbedingungen der Wertstoffe inert sind, Erhitzen der Mischung auf Verdampfungstemperatur unter Vakuum, wobei die Wertstoffe verdampfen, abgezogen und kondensiert werden und der Rückstand als rieselfähiger Feststoff anfällt, wobei die Rückstandslösung auf ein bei Verdampfungstemperatur gehaltenes gerührtes Bett aus körnigem, festem Gut aufgebracht wird. Nachteilig hierbei ist der zusätzliche Einsatz von hoch siedenden Lösungsmitteln, die in einem weiteren Prozess aufgearbeitet werden müssen.

Die Hydrolyse von Isocyanat-Destillationsrückständen mit Wasser zwecks Rückgewinnung des Ausgangsamins, insbesondere bei der Herstellung von TDI, ist ein bereits seit längerer Zeit bearbeitetes Gebiet und beispielsweise in US 3,128,310, US 3,331,876, GB 795,639, DE 27 03 313 A1 und EP 1 935 877 A1 beschrieben. In den zitierten Verfahren wird Isocyanat-Destillationsrückstand bei erhöhtem Druck und erhöhter Temperatur mit Wasser hydrolysiert. Dabei wird ein Teil des Rückstandes in das Ursprungsamin umgewandelt, das nach entsprechender Aufarbeitung wieder in den Phosgenierprozess zurückgeführt werden kann und damit zu einer Rückstandsminimierung führt.

EP 1 413 571 A1 und EP 1 371 633 A1 befassen sich mit der Optimierung der Aufarbeitung von TDI durch Einsatz einer Trennwandkolonne in der Destillation, was unter anderem eine Reduzierung des Gehalts an TDI im Sumpfprodukt zur Folge hat. Aber auch hier kann nicht verhindert werden, dass ein Isocyanat enthaltender Destillationsrückstand anfällt.

WO 2014/009342 A1 befasst sich mit einem Sprühtrocknungsverfahren zur Gewinnung von monomerem Isocyanat (d. h. das als Zielprodukt angestrebte, herzustellende Isocyanat im Unterschied zu unerwünschten hochmolekularen Isocyanatgruppen-haltigen Polymeren) aus Destillationsrückstand enthaltenden Sumpfströmen. Durch die beschriebene Sprühtrocknung werden ein getrockneter, von monomerem Isocyanat weitgehend bis vollständig befreiter Rückstand und ein monomeres Isocyanat umfassender Strom erhalten. Zur Durchführung dieses Verfahrens bedarf es eines speziellen Reaktors, sodass es im Allgemeinen nicht ohne größere Umbauten in eine vorhandene Isocyanat-Produktionsanlage integriert werden kann.

WO 2015/024859 A1 beschreibt ein Verfahren, bei welchem TDI und höher siedende Komponenten einer zweistufigen, kontinuierlichen Destillation zugeführt werden. Auf diese Weise können noch schonendere Bedingungen für die Aufbereitung der organischen Isocyanate eingestellt werden, wodurch die Gefahr unerwünschter Reaktionen der Isocyanate weiter gemindert wird. Zwar kann die Ausbeute an TDI hier maximiert werden, jedoch fallen auch weiterhin Nebenprodukte an.

Die wie zuvor beschriebenen, bei der Herstellung und Aufarbeitung organischer Isocyanate anfallenden Nebenprodukte oder Rückstände werden zumeist der Entsorgung zugeführt. Die Aufgabe der vorliegenden Erfindung besteht darin, die anfallenden Nebenprodukte oder Rückstände als Rohstoffquelle, insbesondere für die Herstellung organischer Isocyanate im Rahmen eines Kreislaufs, zu verwenden. Zu diesem Zweck sollen die anfallenden Nebenprodukte oder Rückstände jeweils effektiv durch eine Verwertung in nutzbare Synthesebausteine als Verwertungsprodukte umgewandelt werden, um Letztere gegebenenfalls nach weiterer Umsetzung, in einen bestehenden Produktionsprozess einzubringen, bevorzugt in die Herstellung organischer Isocyanate zurückzuführen.

Ein erster Gegenstand der Erfindung ist ein Produktionsverfahren zur Herstellung organischer Isocyanate, enthaltend mindestens einen Schritt A zur Herstellung von Phosgen zumindest aus den Reaktanden Chlorgas und Kohlenmonoxid, sowie mindestens einen Schritt B zur Umsetzung zumindest der Reaktanden organisches Amin mit dem in Schritt A gebildeten Phosgen zum organischen Isocyanat als Produkt, dadurch gekennzeichnet, dass in mindestens einem von Schritt A und Schritt B verschiedenen Verwertungsschritt mindestens ein organischer Stoff aus dem Stoffstrom dieses Produktionsverfahrens einer thermischen oxidativen Behandlung unterzogen wird und mindestens ein aus besagtem Verwertungsschritt resultierendes Verwertungsprodukt zur Bereitstellung mindestens eines Reaktanden aus Schritt A und/oder Schritt B genutzt und als solcher dem Produktionsverfahren wieder zugeführt wird.

Ein organischer Stoff ist eine chemische Verbindung, die mindestens eine kovalente Kohlenstoff-Wasserstoff-Bindung im Molekül enthält. Ein organisches Isocyanat ist demnach ein organischer Stoff, der als chemische Verbindung mindestens eine Isocyanatgruppe und mindestens eine kovalente Kohlenstoff-Wasserstoff-Bindung im Molekül enthält. Ein organisches Amin ist *mutatis mutandis* definiert.

In dem erfindungsgemäßen Produktionsverfahren wird im Schritt B organisches Isocyanat als Produkt erhalten. Es ist im Rahmen der vorliegenden Erfindung bevorzugt, wenn in Schritt B als Produkt ein Stoffgemisch, enthaltend organisches Isocyanat, gewonnen wird.

Es sind generell solche erfindungsgemäßen Produktionsverfahren bevorzugt, in denen das in Schritt B gewonnene organische Isocyanat mindestens zwei Isocyanatgruppen enthält. Zu diesem Zweck wird bei der Synthese in Schritt B wiederum bevorzugt als Reaktand organisches Amin mit mindestens zwei Aminogruppen eingesetzt.

Besonders bevorzugt enthält das in Schritt B gewonnene organische Isocyanat mindestens zwei Isocyanatgruppen und weist eine Molmasse von höchstens 1000 g/mol, insbesondere von höchstens 800 g/mol, auf.

Ganz besonders bevorzugte, in Schritt B gewonnene organische Isocyanate werden ausgewählt aus Toluylendiisocyanat (TDI), Methylendi(phenylisocyanat) (MDI) (wiederum bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat (HDI), Isophorondiisocyanat (IPDI), 1,3-Bis(isocyanatomethyl)benzol (XDI), Cyclohexyldiisocyanat (CHDI) oder Mischungen daraus, wobei TDI, MDI oder Mischungen daraus ganz besonders bevorzugte organische Isocyanate sind.

Das erfindungsgemäße Verfahren gewährleistet die Verwertung mindestens eines organischen Stoffes aus dem Stoffstrom zu einem Verwertungsprodukt, das wiederum für die Bereitstellung von Reaktand nutzbar ist. Ein Stoffstrom ist der in einem mehrstufigen chemischen Produktionsverfahren erhaltene Strom von Stoffen, enthaltend Produkt, das jeweils aus einem Verfahrensschritt einer Reaktionsstufe erhalten wird und gegebenenfalls in den nächsten Verfahrensschritt eingeht, und Nebenprodukt, das bei Aufreinigung der Produkte in Form von Reststoff aus Produktgemischen eines Verfahrensschrittes abgetrennt wird. Ein Reststoff kann dabei abgetrenntes Nebenprodukt sein, das in einem Syntheseschritt des Verfahrens gebildet wurde, oder ein bei der Aufreinigung (i.e. Isolierung des Produkts aus dem Stoffstrom) durch chemische Umwandlung des Nebenprodukts oder Produkts entstandener, abgetrennter Stoff, z.B. ein Polymerisat.

Beispielsweise kann der aus dem Sprühtrocknungsverfahren gemäß Druckschrift WO 2014/009342 A1 erhaltene, mindestens einen organischen Stoff enthaltende festförmige Rückstand dem erfindungsgemäßen Verwertungsschritt zugeführt werden.

Als mindestens ein organischer Stoff des besagten Stoffstromes wird bevorzugt mindestens ein zumindest aus dem in Schritt B gewonnenen organischen Isocyanat als Monomer gebildetes Polymer einer thermischen oxidativen Behandlung unterzogen. Dabei wird besonders bevorzugt das besagte Polymer zumindest aus den vorgenannten Ausführungsformen des organischen Isocyanates als Monomer gebildet. Ganz besonders bevorzugte Produktionsverfahren sind dadurch gekennzeichnet, dass das besagte Polymer ein mittleres Molekulargewicht (Mn) von mehr als 1000 g/mol aufweist.

Es ist im Rahmen der vorliegenden Erfindung besonders bevorzugt, wenn die Gesamtmenge der dem mindestens einen Verwertungsschritt zugeführten organischen Stoffe weniger als 50 Gew.-%, insbesondere weniger als 45 Gew.-%, weiter bevorzugt weniger als 40 Gew.-%, ganz besonders bevorzugt weniger als 35 Gew.-%, am bevorzugtesten höchstens 30 Gew.-%, organisches Isocyanat mit mindestens zwei Isocyanatgruppen und einer Molmasse von höchstens 1000 g/mol, insbesondere von höchstens 800 g/mol) enthält.

In einer bevorzugten Ausführungsform des erfindungsgemäßen Verfahrens wird in dem erfindungsgemäßen Verwertungsschritt mindestens ein im Schritt B als Nebenprodukt erhaltener organischer Stoff, besonders bevorzugt mindestens ein im Aufreinigungsschritt C als Reststoff abgetrennter organischer Stoff, im Verwertungsschritt einer thermischen oxidativen Behandlung unterzogen.

Wird, wie zuvor beschrieben, mindestens ein im Aufreinigungsschritt C als Reststoff abgetrennter organischer Stoff im Verwertungsschritt einer thermischen oxidativen Behandlung unterzogen, wird zunächst in mindestens einem Aufreinigungsschritt C aus einem in Schritt B erhaltenen Stoffgemisch zur Aufreinigung des in besagtem Stoffgemisch enthaltenen organischen Isocyanates mindestens ein Reststoff als organischer Stoff des besagten Stoffstromes für die thermische oxidative Behandlung abgetrennt. Dieser Reststoff kann beispielsweise kein organisches Isocyanat, insbesondere kein organisches Isocyanat mit mindestens zwei Isocyanatgruppen, sein.

In einer bevorzugten Ausführungsform des Aufreinigungsschrittes C wird bei der Aufreinigung des in besagtem Stoffgemisch enthaltenen organischen Isocyanates mindestens ein Polymer des gewonnenen organischen Isocyanates (bevorzugt mit einem mittleren Molekulargewicht (Mn) von mindestens 1000 g/mol) als mindestens ein Reststoff abgetrennt, der als organischer Stoff des besagten Stoffstromes im Verwertungsschritt einer thermischen oxidativen Behandlung unterzogen wird.

Im Rahmen einer bevorzugten Ausführungsform der Erfindung wird eine Aufreinigung als mehrstufige destillative Aufarbeitung eines aus Schritt B erhaltenen Stoffgemisches, enthaltend organisches Isocyanat und Lösungsmittel, in reines organisches Isocyanat, reines Lösungsmittel sowie in einen Anteil an Reststoff durchgeführt, die mindestens die folgenden Aufreinigungsschritte C umfasst:
- mehrstufige destillative Aufarbeitung des aus Schritt B erhaltenen Stoffgemisches, enthaltend organisches Isocyanat und Lösungsmittel,
- der aus der mehrstufigen Destillation erhaltene Rückstand wird in einen gerührten und auf 150-280°C beheizten Behälter bei einem Druck von 2 bis 30 mbar geleitet, der mit mindestens einem Kohlenwasserstoff mit einer bei 15 mbar vorliegenden Siedepunktdifferenz zum organischen Isocyanat von mindestens 150 K, in einer Menge von 1 bis 20 Vol.% seines Inhaltes gefüllt ist,
- der Anteil an noch im Rückstand vorhandenen organischen Isocyanates wird abdestilliert,
- der verbleibende Reststoff wird als, bevorzugt rieselfähiger, Feststoff ausgetragen und nach optionaler Mahlung, als für den Verwertungsschritt vorgesehener organischer Stoff des Stoffstromes aus dem Behälter ausgebracht.

Es ist erfindungsgemäß bevorzugt, wenn bei dieser Aufreinigung als besagter Kohlenwasserstoff mit einer bei 15 mbar vorliegenden Siedepunktdifferenz zum organischen Isocyanat von mindestens 150 K Asphalte oder Bitumen oder Mischungen daraus eingesetzt wird.

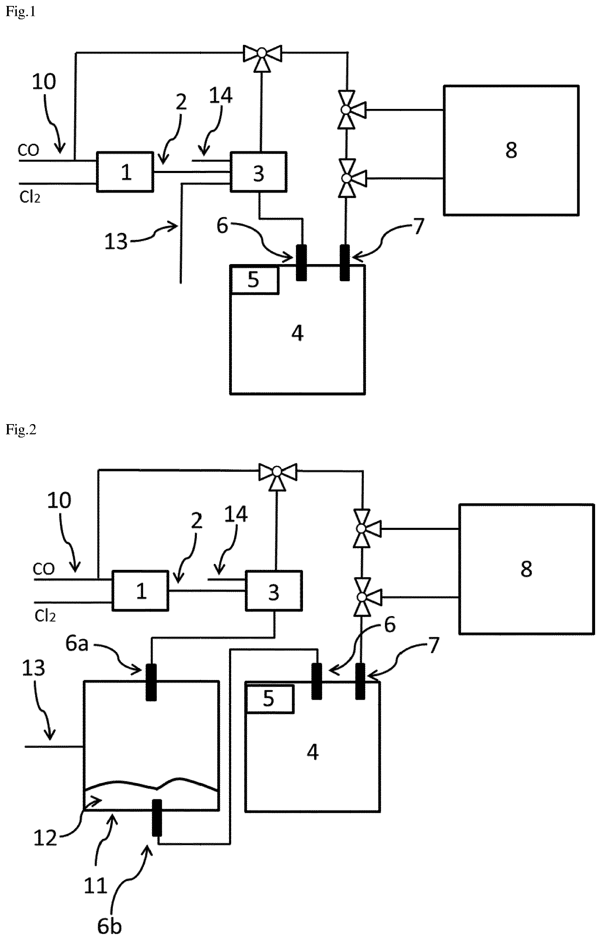

Als Behälter für die Durchführung des mindestens einen Aufreinigungsschrittes C, bevorzugt für die bevorzugte Ausführungsform der vorgenannten Aufreinigung als mehrstufige destillative Aufarbeitung, wird besonders bevorzugt ein rührbarer Sumpfbehälter, an dem sich ein Kondensationssystem anschließt ausgewählt. Ein solcher Sumpfbehälter wird beispielsweise in Fig.2 als Sumpfbehälter (11) mit enthaltenem Sumpf (12) als organischem Stoff des besagten Stoffstromes abgebildet.

Als Behälter für die Durchführung des mindestens einen Aufreinigungsschrittes C, bevorzugt für die bevorzugte Ausführungsform der vorgenannten Aufreinigung als mehrstufige destillative Aufarbeitung, eignet sich ebenso mindestens ein beheizter, rückstandumschaufelnder Vakuumtrockner mit horizontaler Welle, wie er in der Druckschrift EP 0 626 368 A1 beschrieben wird.

Ferner ist es im Rahmen einer Ausführungsform möglich, eine Aufreinigung eines aus Schritt B erhaltenen Stoffgemisches, enthaltend organisches Isocyanat und Lösungsmittel, in reines organisches Isocyanat, reines Lösungsmittel sowie in einen Anteil an Reststoff durchzuführen, die mindestens die folgenden Aufreinigungsschritte C umfasst:
- mehrstufige destillative Aufarbeitung des aus Schritt B erhaltenen Stoffgemisches, enthaltend organisches Isocyanat und Lösungsmittel,
- Einleiten des aus der mehrstufigen Destillation erhaltenen Rückstandes in einen gerührten und auf eine Temperatur über der Siedetemperatur des organischen Isocyanates beheizten Behälter, enthaltend ein Wirbelbett aus körnigem Feststoff, und Abdestillieren von noch im eingeleiteten Rückstand vorhandenem, organischem Isocyanat,
- der verbleibende Reststoff wird als, bevorzugt rieselfähiger Feststoff ausgetragen und nach optionaler Mahlung, als für den Verwertungsschritt vorgesehener organischer Stoff des Stoffstromes aus dem Behälter ausgebracht.

Als körniger Feststoff zur Bereitstellung des Wirbelbettes eignet sich bevorzugt ein Feststoff aus Partikeln mit einem durchschnittlichen Teilchendurchmesser im Bereich von 0,1 bis 5 mm, stärker bevorzugt im Bereich von 0,5 bis 4 mm und am stärksten bevorzugt im Bereich von 0,5 bis 3 mm Als körniger Feststoff wird vorzugsweise ein Teil des Rückstandes eingesetzt, der bei einer vorangegangenen Aufreinigung des Produktgemisches aus Schritt B als Rückstand gewonnen wurde.

Für die Ausführung dieser Aufreinigungsvariante und insbesondere für die Bereitstellung des Wirbelbettes, wird ausdrücklich auf die Druckschrift EP 2 540 702 A2 verwiesen, auf deren Offenbarung hiermit explizit vollumfänglich Bezug genommen wird.

Das erfindungsgemäße Produktionsverfahren umfasst mindestens einem von Schritt A und Schritt B verschiedenen Verwertungsschritt, in dem mindestens ein organischer Stoff aus dem Stoffstrom dieses Produktionsverfahrens einer thermischen oxidativen Behandlung unterzogen wird. Besagter organischer Stoff des besagten Stoffstromes ist bevorzugt ein Feststoff, insbesondere festförmiger Sumpf einer Aufreinigung mit mindestens einem Aufreinigungsschritt C.

Unter einer thermischen oxidativen Behandlung wird die Zuführung von Wärme in Gegenwart mindestens eines Oxidationsmittels, bevorzugt in Gegenwart von zumindest Sauerstoffgas als Oxidatiosmittel, verstanden.

Die im Schritt B des erfindungsgemäßen Produktionsverfahrens oft als Feststoff, insbesondere als polymerer Feststoff, anfallenden organischen Stoffe des Stoffstroms, insbesondere Reststoff des Aufreinigungsschritts C, werden, bevorzugt unter Zugabe von Sauerstoffgas, zu Kohlenwasserstoffen mit einem Siedepunkt von unter 100°C bei 1013 mbar und/oder zu weiteren Synthesegasbestandteilen als Verwertungsprodukt umgewandelt. Der Prozess wird dabei bevorzugt so gesteuert, dass mindestens eine Verbindung aus CO, CO₂, H₂O, CH₄, HCN, H₂, N₂, NxOy oder Mischungen daraus als Hauptbestandteil anfällt. Unter NxOy werden insbesondere N₂O, NO, NO₂, N₂O₅ oder Mischungen daraus verstanden. Weitere Bestandteile können neben höheren Kohlenwasserstoffen (Aromaten, Ethylen, Propylen etc.) auch Spezies sein, welche aus der Anlage ausgetragen werden (bspw. Metallcarbonyle) oder als Verunreinigung im Nebenproduktstrom anfallen (bspw. Chlor-haltige Verbindungen).

Die erfindungsgemäße thermische, oxidative Umwandlung ist bevorzugterweise eine partielle Oxidation (d.h. der dem Fachmann als "POX" bekannten partiellen Oxidation), der in dem im Schritt B des erfindungsgemäßen Produktionsverfahrens, bevorzugt als Feststoff, insbesondere als polymerer Feststoff, anfallenden organischen Stoffe des Stoffstroms, insbesondere Reststoff des Aufreinigungsschritts C, unter Verwendung von Sauerstoffgas enthaltendem Gas als Oxidationsmittel wobei mindestens ein Parameter, ausgewählt aus Sauerstoffgasgehalt des Oxidationsmittels, Sauerstoffgasdruck während der oxidativen Behandlung in der Reaktionszone, Gesamtdruck während der oxidativen Behandlung in der Reaktionszone, Temperatur während der oxidativen Behandlung in der Reaktionszone und Verweilzeit besagter resultierender Verwertungsprodukte (bevorzugt Kohlenmonoxid) in der Reaktionszone der oxidativen Behandlung geregelt wird.

Die Herstellung von Synthesegas aus Kohlenwasserstoffen durch Oxidation ist allgemein bekannt. WO 2007/134727 A1 beschreibt ein Verfahren zur Herstellung von Synthesegas, bei welchem ein autothermer Reformer zur Gewinnung von Synthesegas eingesetzt wird. Dabei wird ein sauerstoffreicher Gasstrom in Gegenwart eines Spaltkatalysators autotherm zu einem Synthesegas reformiert.

DE102006045379B4 beschreibt ein Verfahren zur Herstellung von kohlenwasserstoffhaltigen Edukten bei welchem das Ausgangsmaterial unter Zusatz von sauerstoffhaltigem Gas und Wasserstoff bei Temperaturen von 1200 bis 1500°C und Drücken von 15 bis 100 bar (absolut) durch nichtkatalytische partielle Oxidation autotherm zu einem als Hauptkomponenten H₂ und Kohlenmonoxid (CO) sowie die Komponenten CO₂, H₂O, CH₄ und Spuren von H₂S, COS, CxHy, N₂ und Ar enthaltendem Rohgas vergast und anschließend das in dem Rohgas enthaltene CO unter Zusatz von Wasserdampf zu CO₂ und weiterem H₂ konvertiert wird.

Ein "Verwertungsprodukt" gemäß vorliegender Erfindung ist eine chemische Verbindung, die als Reaktand mindestens eines Schrittes A und/oder Schrittes B des erfindungsgemäßen Verfahrens fungieren kann oder die für die Herstellung eines solchen vorgenannten Reaktanden genutzt werden kann.

Ein erfindungsgemäß bevorzugtes Verwertungsprodukt ist bei 20°C und 1013 mbar gasförmig.

Bevorzugterweise wird in dem erfindungsgemäßen Verfahren nach der thermischen oxidativen Behandlung des Verwertungsschrittes mindestens ein Verwertungsprodukt ausgewählt aus CO₂, CO, H₂, HCN, H₂O, NH₃ (besonders bevorzugt mindestens ein Verwertungsprodukt ausgewählt aus CO₂, CO, H₂, H₂O) erhalten.

Ganz besonders bevorzugt ist ein erfindungsgemäßes Verfahren das sich dadurch gekennzeichnet, dass mindestens Kohlenmonoxid als Verwertungsprodukt erhalten wird und dieses als Reaktand dem Schritt A des Verfahrens zugeführt wird. Durch dieses Vorgehen wird zumindest ein Teil des dem Schritt A zugeführten Kohlenmonoxidgases (CO) durch das Verwertungsprodukt beigesteuert. Der CO Primärbedarf der Isocyanatproduktion kann somit folglich verringert werden.

Mit Bezug auf diese ganz besonders bevorzugte Ausführungsform werden die Reaktionsparameter (insbesondere Reaktionsparameter, ausgewählt aus Sauerstoffgasgehalt des Oxidationsmittels, Sauerstoffgasdruck während der oxidativen Behandlung in der Reaktionszone, Gesamtdruck während der oxidativen Behandlung in der Reaktionszone, Temperatur während der oxidativen Behandlung in der Reaktionszone und Verweilzeit besagter resultierender Verwertungsprodukte (bevorzugt Kohlenmonoxid) in der Reaktionszone oder Mischungen daraus) der erfindungsgemäßen thermischen, oxidativen Behandlung wiederum bevorzugt so angepasst, dass bei besagter thermischer, oxidativer Behandlung eine maximale Ausbeute an CO erhalten wird. Besonders bevorzugt nach erfolgter Aufreinigung wird das CO der Phosgenherstellung im Schritt A zugeführt. Das unter Verbrauch von Chlor gebildete Phosgen wird anschließend genutzt, um dieses mit organischem Amin zu Isocyanaten umzusetzen.

In Folge der Wahl von Sauerstoff als bevorzugtes Oxidationsmittel, wird bei der thermischen, oxidativen Behandlung ein signifikanter Anteil des im organischen Stoff aus dem Stoffstrom dieses Produktionsverfahrens (bevorzugt im organischen Stoff aus dem Stoffstrom des TDI-Produktionsverfahrens), insbesondere des im Reststoff aus Aufreinigungsschritt C, enthaltenen Kohlenstoffes zum thermodynamisch bevorzugten CO₂ umgesetzt werden. Durch den Einsatz von RWGS oder CO₂-Elektrolyse kann das erhaltene CO₂ wieder zurück zu CO umgewandelt werden. Das bei der CO₂-Elektrolyse an der Anode gewonnene Sauerstoffgas wird bevorzugterweise der thermischen, oxidativen Behandlung als Oxidationsmittel zugeführt. Je nach Bedarf kann die anodische Reaktion der Elektrolyse auch für alternative elektrochemische Oxidation benutzt werden bspw. für die Herstellung von oxidierten Kohlenwasserstoffen oder von Chlor. Alternativ kann das bei der thermischen, oxidativen Behandlung erhaltene CO₂ auch durch Hydrierung zu Methanol umgesetzt werden. Technische Prozesse dafür sind allgemein bekannt. Der CO₂ Feed für vorgenannte Umwandlungsmethoden ist in jedem Fall entsprechend aufzureinigen, sodass keine Katalysatorgifte eingebracht werden.

Es ist erfindungsgemäß bevorzugt, CO₂ als erhaltenes Verwertungsprodukt elektrochemisch in CO als Reaktanden des Schrittes A umzuwandeln. Die elektrochemische Gewinnung von CO aus CO₂ unter Bildung von O₂ ist allgemein beschrieben. Nature Catalysis Vol. 1, 32-39 (2018) beschreibt bspw. eine Gasdiffusionselektrode (GDE) zur Reduktion von Kohlendioxid auf Basis von porösem Silberpulver als Elektrokatalysator und deren Verwendung als für die elektrochemische Reduktion von Kohlendioxid (CO₂) zu CO bei technisch relevanten Stromdichten.

Es ist erfindungsgemäß bevorzugt, CO₂ als erhaltenes Verwertungsprodukt mittels Reverse-Water-Gas-Shift (RWGS) in CO als Reaktanden des Schrittes A umzuwandeln. Auch die Nutzung der RWGS Reaktion ist allgemein beschrieben. Hier wird unter Nutzung von Wasserstoff CO₂ über einem Katalysator zu CO umgesetzt. Quelle des Wasserstoffs kann hier bspw. eine Wasserelektrolyse sein.

Je nach nachfolgendem Produktionsschritt sind die Synthesegasbestandteile weiter aufzureinigen. So müssen bspw. für die Ammoniaksynthese sämtliche CO₂ Anteile entfernt werden, was üblicherweise durch Scrubbing-Prozesse erfolgt. Spuren von CO werden hier über Methanisierung oder N₂-scrubbing entfernt. Zusätzlich können je nach Bedarf auch Druckwechseladsorptionen zur weiteren Aufreinigung eingesetzt werden.

In Abhängigkeit der verwendeten Reaktionsparameter kann die Selektivität zu einzelnen Bestandteilen angepasst werden. Zur Steuerung der Produktverteilung kann auch ein heterogener oder homogener Katalysator eingesetzt werden.

Die einzelnen Bestandteile eines bei der erfindungsgemäßen thermischen, oxidativen Behandlung erhaltenen Produktgases können durch den Einsatz technisch verfügbarer Auftrennungsverfahren getrennt und aufgereinigt werden. Einzelne Bestandteile können dabei direkt genutzt werden (CO, HCN, H₂, höhere Kohlenwasserstoffe (KW) etc.), andere benötigen eine weitere Aufarbeitung (bspw. CO₂, NOx).

Weitere Bestandteile des bei der erfindungsgemäßen thermischen, oxidativen Behandlung erhaltenen Produktgases können verschiedenen Zwecken zugeführt werden. H₂ bspw. kann zur Produktion von Aminen eingesetzt werden. CH₄ kann als Edukt in einen Steamreformer überführt werden und so zu CO ungewandelt werden. NyOx kann ebenfalls aufgebarbeitet werden oder unter Nutzung eines DENOX Katalysators in N₂ überführt und anschließend entsorgt werden. NH₃ kann nach Oxidation zu HNO₃ für die Nitrierung von Aromaten verwendet werden. HCN kann zur Herstellung von organischen Diaminen verwendet werden, welche letztendlich wieder als Reaktand im Schritt B zu Isocyanaten umgesetzt werden können.

Weitere Bestandteile des Produktgases oder andere Nebenprodukte (bspw. Asche/Schlacke) können weiter aufbereitet werden oder werden der Entsorgung zugeführt.

Allen Bestandteilen des Produktgases ist dabei gemein, dass nach erfolgter Auftrennung die jeweiligen Bestandteile mit weiteren Komponenten vermengt werden können, um so die gewünschte Zusammensetzung des Synthesegases zu erreichen.

Ein weiterer Gegenstand der Erfindung ist eine Produktionsanlage zur Herstellung organischer Isocyanate, umfassend eine Produktionseinheit zur Herstellung von Phosgen aus Chlor und Kohlenmonoxid, die in gegebenenfalls absperrbarer Fluidverbindung für den Phosgentransfer mit einer Produktionseinheit für organisches Isocyanat steht, dadurch gekennzeichnet, dass ferner eine Verwertungseinheit mit mindestens einer Heizquelle, mindestens einem Stoffzufluss und mindestens einem Verwertungsproduktabfluss vorhanden ist, wobei der Stoffzufluss mit der Verwertungseinheit und der Produktionseinheit in gegebenenfalls absperrbarer Fluidverbindung steht, wodurch ein organischen Stoff aus einem Stoffstrom der Produktionsanlage in die Verwertungseinheit eingebracht werden kann und darin durch Wärmeeintrag mittels Heizquelle thermisch oxidativ behandelt werden kann und das resultierende Verwertungsprodukt durch den Verwertungsproduktabfluss aus der Verwertungseinheit gegebenenfalls nach Durchlauf mindestens einer Umsetzungsstufe als Reaktand in die Produktionseinheit oder in die Produktionseinheit für organisches Isocyanat eingebracht werden kann.

Unter einer Fluidverbindung wird eine Vorrichtung der Produktionsanlage verstanden, die Anlagenteile miteinander verbindet und durch die sich ein Stoff, der in jedem Aggregatzustand vorliegen kann, von einem Anlagenteil zum nächsten transportieren lässt, beispielsweise eine Zuleitung in Form eines Rohres.

Die Produktionseinheit für organisches Isocyanat umfasst weiterhin einen Stoffzufluss für organisches Amin.

Die Verwertungseinheit der Produktionsanlage weist bevorzugt einen weiteren Stoffzufluss für Oxidationsmittel, bevorzugt für sauerstoffgas-haltiges Gas, insbesondere für Sauerstoffgas, auf. Dieser Stoffzufluss enthält weiter bevorzugt eine Regeleinheit zur Regelung der in die Verwertungseinheit einzuspeisenden Menge an Oxidationsmittel.

Eine bevorzugte Produktionsanlage ist dadurch gekennzeichnet, dass der Verwertungsproduktabfluss mit der Produktionseinheit des Phosgens in gegebenenfalls absperrbarer Fluidverbindung, insbesondere mit der Kohlenmonoxidzuführung der Produktionseinheit des Phosgens, steht. Dabei kann das Kohlenmonoxid als Verwertungsprodukt vor der Zuführung in die Kohlenmonoxidzuführung der Produktionseinheit des Phosgens in einer Aufreinigungsvorrichtung aufgearbeitet werden.

In einer bevorzugten Ausführungsform der erfindungsgemäßen Produktionsanlage steht die Produktionseinheit für organisches Isocyanat über den Stoffzufluss zunächst mit einer Isocyanat-Aufreinigungs-Einheit in gegebenenfalls absperrbarer Fluidverbindung, wobei der Sumpf der Isocyanat-Aufreinigungs-Einheit in gegebenenfalls absperrbarer Fluidverbindung mit dem Stoffzufluss der Verwertungseinheit steht.

In Fig.1 und Fig.2 werden folgende Zeichen verwendet:
- 1: Produktionseinheit zur Herstellung von Phosgen aus Chlor und Kohlenmonoxid
- 2: Fluidverbindung für den Phosgentransfer, optional absperrbar
- 3: Produktionseinheit für organisches Isocyanat
- 4: Verwertungseinheit
- 5: Heizquelle
- 6: Stoffzufluss
- 6a: Stoffzufluss
- 6b: Abfluss für organischen Stoff des Stoffstroms
- 7: Verwertungsproduktabfluss
- 8: Umsetzungsstufe zur Umsetzung von Verwertungsprodukt in Reaktand für die Produktionseinheit 1 oder Produktionseinheit 3
- 9: Fluidverbindung für Verwertungsprodukt, gegebenenfalls absperrbar
- 10: Kohlenmonoxidzuführung
- 11: Isocyanat-Aufreinigungs-Einheit
- 12: Sumpf
- 13: Produktableitung für organisches Isocyanat
- 14: Stoffzufluss für organisches Amin

In Fig. 1 wird ein Beispiel für eine Ausführungsform einer Produktionsanlage zur Herstellung organischer Isocyanate gegeben, umfassend eine Produktionseinheit (1) zur Herstellung von Phosgen aus Chlor und Kohlenmonoxid, die in gegebenenfalls absperrbarer Fluidverbindung (2) für den Phosgentransfer mit einer Produktionseinheit für organisches Isocyanat (3) steht, dadurch gekennzeichnet, dass ferner eine Verwertungseinheit (4) mit mindestens einer Heizquelle (5), mindestens einem Stoffzufluss (6) und mindestens einem Verwertungsproduktabfluss (7) vorhanden ist, wobei der Stoffzufluss (6) mit der Verwertungseinheit (4) und der Produktionseinheit (3) in gegebenenfalls absperrbarer Fluidverbindung steht, wodurch ein organischer Stoff aus einem Stoffstrom der Produktionsanlage in die Verwertungseinheit (4) eingebracht werden kann und darin durch Wärmeeintrag mittels Heizquelle (5) thermisch oxidativ behandelt werden kann und das resultierende Verwertungsprodukt durch den Verwertungsproduktabfluss (7) aus der Verwertungseinheit (4) gegebenenfalls nach Durchlauf mindestens einer Umsetzungsstufe (8) als Reaktand in die Produktionseinheit (1) oder in die Produktionseinheit für organisches Isocyanat (3) eingebracht werden kann.

Dabei ist es bevorzugt, wenn der Verwertungsproduktabfluss (7) mit der Produktionseinheit (1) in gegebenenfalls absperrbarer Fluidverbindung (9), insbesondere mit der Kohlenmonoxidzuführung (10) der Produktionseinheit (1), steht. Zur Aufarbeitung des Verwertungsproduktes Kohlenmonoxid, kann das Verwertungsprodukt vor der Zuführung in die Kohlenmonoxidzuführung (10) in einer Aufreinigungseinheit (nicht abgebildet) aufgearbeitet werden.

In Fig. 2 wird ein Beispiel für eine Ausführungsform einer Produktionsanlage zur Herstellung organischer Isocyanate gegeben, die im Veregleich zur Anlage der Fig.1 zusätzlich eine Isocyanat-Aufreinigungs-Einheit (11) enthält. Dabei steht die Produktionseinheit für organisches Isocyanat (3) über den Stoffzufluss (6a) zunächst mit einer Isocyanat-Aufreinigungs-Einheit (11) in gegebenenfalls absperrbarer Fluidverbindung, wobei der Sumpf (12) der Isocyanat-Aufreinigungs-Einheit (11) über einen Abfluss (6b) für organischen Stoff des Stoffstroms in gegebenenfalls absperrbarer Fluidverbindung mit dem Stoffzufluss (6) der Verwertungseinheit (4) steht.

Sollte für die Durchführung der thermisch oxidativen Behandlung in der Verwertungseinheit (4) die Zufuhr von zusätzlichem Oxidationsmittel notwendig sein, so kann die Verwertungseinheit (4) mit einem Zufluss für Oxidationsmittel ausgestattet sein.

Die Erfindung wird nachfolgend durch die Aspekte 1 bis 17 sowie durch die Beispiele, welche jedoch keine Beschränkung der Erfindung darstellen, näher erläutert.
1. Produktionsverfahren zur Herstellung organischer Isocyanate, enthaltend mindestens einen Schritt A zur Herstellung von Phosgen zumindest aus den Reaktanden Chlorgas und Kohlenmonoxid, sowie mindestens einen Schritt B zur Umsetzung zumindest der Reaktanden organisches Amin mit dem in Schritt A gebildeten Phosgen zum organischen Isocyanat als Produkt, dadurch gekennzeichnet, dass in mindestens einem von Schritt A und Schritt B verschiedenen Verwertungsschritt mindestens ein organischer Stoff aus dem Stoffstrom dieses Produktionsverfahrens einer thermischen oxidativen Behandlung unterzogen wird und mindestens ein aus besagtem Verwertungsschritt resultierendes Verwertungsprodukt zur Bereitstellung mindestens eines Reaktanden aus Schritt A und/oder Schritt B genutzt und dem Produktionsverfahren als solcher wieder zugeführt wird.
2. Verfahren nach Aspekt 1, dadurch gekennzeichnet, dass in Schritt B als Produkt ein Stoffgemisch, enthaltend organisches Isocyanat, gewonnen wird.
3. Verfahren nach einem der Aspekte 1 oder 2, dadurch gekennzeichnet, dass das in Schritt B gewonnene organische Isocyanat mindestens zwei Isocyanatgruppen enthält.
4. Verfahren nach einem der vorangehenden Aspekte, dadurch gekennzeichnet, dass das in Schritt B gewonnene organische Isocyanat eine Molmasse von höchstens 1000 g/mol, insbesondere von höchstens 800 g/mol, aufweist.
5. Verfahren nach einem der vorangehenden Aspekte, dadurch gekennzeichnet, dass als mindestens ein organischer Stoff des besagten Stoffstromes mindestens ein zumindest aus dem in Schritt B gewonnenen organischen Isocyanat als Monomer gebildetes Polymer einer thermischen oxidativen Behandlung unterzogen.
6. Verfahren nach Aspekt 5, dadurch gekennzeichnet, dass das besagte Polymer ein mittleres Molekulargewicht (Mn) von mindestens 1000 g/mol aufweist.
7. Verfahren nach Aspekt 2, dadurch gekennzeichnet, dass nach Schritt B zur Aufreinigung des in besagtem Stoffgemisch enthaltenen organischen Isocyanates in mindestens einem Aufreinigungsschritt C aus dem in Schritt B erhaltenen Stoffgemisch mindestens ein Reststoff als organischer Stoff des besagten Stoffstromes für die thermische oxidative Behandlung abgetrennt wird, der bevorzugt kein organisches Isocyanat, insbesondere der kein organisches Isocyanat mit mindestens zwei Isocyanatgruppen ist.
8. Verfahren nach Aspekt 2 oder 7, dadurch gekennzeichnet, dass nach Schritt B zur Aufreinigung des in besagtem Stoffgemisch enthaltenen organischen Isocyanates in mindestens einem Aufreinigungsschritt C aus dem in Schritt B erhaltenen Stoffgemisch mindestens ein Polymer nach einem der Aspekte 5 oder 6 als mindestens ein Reststoff abgetrennt wird, der als organischer Stoff des besagten Stoffstromes im Verwertungsschritt einer thermischen oxidativen Behandlung unterzogen wird.
9. Verfahren nach einem der Aspekte 7 der 8, dadurch gekennzeichnet, dass zur Aufreinigung eine mehrstufige destillative Aufarbeitung eines aus Schritt B erhaltenen Stoffgemisches, enthaltend organisches Isocyanat und Lösungsmittel, in reines organisches Isocyanat, reines Lösungsmittel sowie in einen Anteil an Reststoff durchgeführt wird, die mindestens die folgenden Aufreinigungsschritte C umfasst:
   - mehrstufige destillative Aufarbeitung des aus Schritt B erhaltenen Stoffgemisches, enthaltend organisches Isocyanat und Lösungsmittel,
   - der aus der mehrstufigen Destillation erhaltene Rückstand wird in einen gerührten und auf 150-280°C beheizten Behälter (bevorzugt auf 150-280°C beheizten Sumpfbehälter) bei einem Druck von 2-30 mbar geleitet, der mit mindestens einem Kohlenwasserstoff mit einer bei 15 mbar vorliegenden Siedepunktdifferenz zum organischen Isocyanat von mindestens 150 K, in einer Menge von 1 bis 20 Vol.% seines Inhaltes gefüllt ist,
   - der Anteil an noch im Rückstand vorhandenen organischen Isocyanates wird abdestilliert,
   - der verbleibende Reststoff wird als Feststoff aus dem Behälter als besagter organischer Stoff des besagten Stoffstroms ausgebracht und optional gemahlen.
10. Verfahren nach einem der Aspekte 1 bis 9, dadurch gekennzeichnet, dass es sich bei dem organischen Stoff des besagten Stoffstromes um einen Feststoff, insbesondere um den Sumpf des Aufreinigungsschrittes C gemäß Aspekt 7 oder 8, handelt.
11. Verfahren nach einem der Aspekte 1 bis 10 dadurch gekennzeichnet, dass das nach der thermischen oxidativen Behandlung des Verwertungsschrittes erhaltene Verwertungsprodukt bei 20°C und 1013 mbar gasförmig ist.
12. Verfahren nach einem der Aspekte 1 bis 11 dadurch gekennzeichnet, dass nach der thermischen oxidativen Behandlung des Verwertungsschrittes mindestens ein Verwertungsprodukt ausgewählt aus CO₂, CO, H₂, H₂O erhalten wird.
13. Verfahren nach einem der Aspekte 1 bis 11, dadurch gekennzeichnet, dass mindestens Kohlenmonoxid als Verwertungsprodukt erhalten wird und dieses als Reaktand dem Schritt A des Verfahrens zugeführt wird.
14. Produktionsanlage zur Herstellung organischer Isocyanate, mindestens umfassend eine Produktionseinheit zur Herstellung von Phosgen aus Chlor und Kohlenmonoxid, die in gegebenenfalls absperrbarer Fluidverbindung für den Phosgentransfer mit einer Produktionseinheit für organisches Isocyanat steht, dadurch gekennzeichnet, dass ferner eine Verwertungseinheit mit mindestens einer Heizquelle, mindestens einem Stoffzufluss und mindestens einem Verwertungsproduktabfluss vorhanden ist, wobei der Stoffzufluss mit der Verwertungseinheit und der Produktionseinheit in gegebenenfalls absperrbarer Fluidverbindung steht, wodurch ein organischer Stoff aus einem Stoffstrom der Produktionsanlage in die Verwertungseinheit eingebracht werden kann und darin durch Wärmeeintrag mittels Heizquelle thermisch oxidativ behandelt werden kann und das resultierende Verwertungsprodukt durch den Verwertungsproduktabfluss aus der Verwertungseinheit gegebenenfalls nach Durchlauf mindestens einer Umsetzungsstufe als Reaktand in die Produktionseinheit oder in die Produktionseinheit für organisches Isocyanat eingebracht werden kann.
15. Produktionsanlage nach Aspekt 14 dadurch gekennzeichnet, dass der Verwertungsproduktabfluss mit der Produktionseinheit für Phosgen in gegebenenfalls absperrbarer Fluidverbindung, insbesondere mit der Kohlenmonoxidzuführung der Produktionseinheit für Phosgen, steht.
16. Produktionsanlage nach einem der Ansprüche 14 oder 15, dadurch gekennzeichnet, dass die Verwertungseinheit einen Zufluss für Oxidationsmittel, bevorzugt für sauerstoffgas-haltiges Gas, insbesondere für Sauerstoffgas, aufweist.
17. Produktionsanlage nach einem der Aspekte 14 oder 15 dadurch gekennzeichnet, dass die Produktionseinheit für organisches Isocyanat über den Stoffzufluss zunächst mit einer Isocyanat-Aufreinigungs-Einheit in gegebenenfalls absperrbarer Fluidverbindung steht, wobei der Sumpf der Isocyanat-Aufreinigungs-Einheit über einen Abfluss für organischen Stoff des Stoffstroms in gegebenenfalls absperrbarer Fluidverbindung mit dem Stoffzufluss der Verwertungseinheit steht.

### Beispiele

### Beispiel 1 (Erfindungsgemäß)

Ein typischer fester Produktionsrückstand wie er üblicherweise in einem analog zu EP 0 548 685 A2 oder EP 0 626 368 A1 ausgeführten Verfahren hergestellt wird wurde nach DIN EN ISO 5959-2 vermessen. Dazu wurde in einer Aluschale mit den Abmessungen 75mm x 75mm eine 2mm hohe Schichtdicke eingefüllt. Das Pulver wurde dann für 8 Minuten einer Bestrahlungsstärke von 25 bzw. 50 kW/m² in Abwesenheit einer Zündflamme ausgesetzt. Die Konzentration verschiedener Komponenten wurde per FT-IR nach 4 und 8 Minuten bestimmt. Weitere organische Komponenten wie bspw. Benzol, Toluol etc. erfolgte durch den Einsatz speziell präparierte Filter, welche analytisch ausgewertet wurden. Es wurden folgenden Konzentrationen bestimmt:

### 25 mW/m2 Bestrahlungsstärke:

| **Komponente** | **Konzentration nach 4 min / ppm** | **Konzentration nach 8 min / ppm** |
|---|---|---|
| CO₂ | 365 | 751 |
| CO | 12 | 42 |
| HCN | - | - |
| NH₃ | - | - |

### 50 mW/m2 Bestrahlungsstärke:

| **Komponente** | **Konzentration nach 4 min / ppm** | **Konzentration nach 8 min / ppm** |
|---|---|---|
| CO₂ | 2023 | 2891 |
| CO | 152 | 369 |
| CN | 62 | 191 |
| NH₃ | 398 | 533 |

Die als Komponente erhaltenen Produkte der thermischen Behandlung waren für die Bereitstellung von Reaktanden für das erfindungsgemäße Produktionsverfahren geeignet, beispielsweise eignete sich das CO für die Phosgensynthese als Reaktand zur Herstellung von TDI als organisches Isocyanat durch Umsetzung von Phosgen mit TDA.

## Patentansprüche

1. Produktionsverfahren zur Herstellung organischer Isocyanate, enthaltend mindestens einen Schritt A zur Herstellung von Phosgen zumindest aus den Reaktanden Chlorgas und Kohlenmonoxid, sowie mindestens einen Schritt B zur Umsetzung zumindest der Reaktanden organisches Amin mit dem in Schritt A gebildeten Phosgen zum organischen Isocyanat als Produkt, **dadurch gekennzeichnet, dass** in mindestens einem von Schritt A und Schritt B verschiedenen Verwertungsschritt mindestens ein organischer Stoff aus dem Stoffstrom dieses Produktionsverfahrens einer thermischen oxidativen Behandlung unterzogen wird und mindestens ein aus besagtem Verwertungsschritt resultierendes Verwertungsprodukt zur Bereitstellung mindestens eines Reaktanden aus Schritt A und/oder Schritt B genutzt und als solcher dem Produktionsverfahren wieder zugeführt wird.

2. Verfahren nach Anspruch 1, **dadurch gekennzeichnet, dass** in Schritt B als Produkt ein Stoffgemisch, enthaltend organisches Isocyanat, gewonnen wird.

3. Verfahren nach einem der Ansprüche 1 oder 2, **dadurch gekennzeichnet, dass** das in Schritt B gewonnene organische Isocyanat mindestens zwei Isocyanatgruppen enthält.

4. Verfahren nach einem der vorangehenden Ansprüche, **dadurch gekennzeichnet, dass** das in Schritt B gewonnene organische Isocyanat eine Molmasse von höchstens 1000 g/mol, insbesondere von höchstens 800 g/mol, aufweist.

5. Verfahren nach einem der vorangehenden Ansprüche, **dadurch gekennzeichnet, dass** als mindestens ein organischer Stoff des besagten Stoffstromes mindestens ein zumindest aus dem gewonnenen organischen Isocyanat als Monomer gebildetes Polymer einer thermischen oxidativen Behandlung unterzogen.

6. Verfahren nach Anspruch 5, **dadurch gekennzeichnet, dass** das besagte Polymer ein mittleres Molekulargewicht (Mn) von mehr als 1000 g/mol aufweist.

7. Verfahren nach Anspruch 2, **dadurch gekennzeichnet, dass** nach Schritt B zur Aufreinigung des in besagtem Stoffgemisch enthaltenen organischen Isocyanates in mindestens einem Aufreinigungsschritt C aus dem in Schritt B erhaltenen Stoffgemisch mindestens ein Reststoff als organischer Stoff des besagten Stoffstromes für die thermische oxidative Behandlung abgetrennt wird, der bevorzugt kein organisches Isocyanat, insbesondere der kein organisches Isocyanat mit mindestens zwei Isocyanatgruppen, ist.

8. Verfahren nach Anspruch 2 oder 7, **dadurch gekennzeichnet, dass** nach Schritt B zur Aufreinigung des in besagtem Stoffgemisch enthaltenen organischen Isocyanates in mindestens einem Aufreinigungsschritt C aus dem in Schritt B erhaltenen Stoffgemisch mindestens ein Polymer nach einem der Ansprüche 5 oder 6 als mindestens ein Reststoff abgetrennt wird, der als organischer Stoff des besagten Stoffstromes im Verwertungsschritt einer thermischen oxidativen Behandlung unterzogen wird.

9. Verfahren nach einem der Ansprüche 7 der 8, **dadurch gekennzeichnet, dass** zur Aufreinigung eine mehrstufige destillative Aufarbeitung eines aus Schritt B erhaltenen Stoffgemisches, enthaltend organisches Isocyanat und Lösungsmittel, in reines organisches Isocyanat, reines Lösungsmittel sowie in einen Anteil an Reststoff durchgeführt wird, die mindestens die folgenden Aufreinigungsschritte C umfasst:
- mehrstufige destillative Aufarbeitung des aus Schritt B erhaltenen Stoffgemisches, enthaltend organisches Isocyanat und Lösungsmittel,
- der aus der mehrstufigen Destillation erhaltene Rückstand wird in einen gerührten und auf 150-280°C beheizten Behälter bei einem Druck von 2 bis 30 mbar geleitet, der mit mindestens einem Kohlenwasserstoff mit einer bei 15 mbar vorliegenden Siedepunktdifferenz zum organischen Isocyanat von mindestens 150 K, in einer Menge von 1 bis 20 Vol.% seines Inhaltes gefüllt ist,
- der Anteil an noch im Rückstand vorhandenen organischen Isocyanates wird abdestilliert,
- der verbleibende Reststoff wird als Feststoff aus dem Behälter als besagter organischer Stoff des besagten Stoffstroms ausgebracht und optional gemahlen.

10. Verfahren nach einem der Ansprüche 1 bis 9, **dadurch gekennzeichnet, dass** es sich bei dem organischen Stoff des besagten Stoffstromes um einen Feststoff, insbesondere um den festförmigen Sumpf des Aufreinigungsschrittes C gemäß einem der Ansprüche 7 bis 9, handelt.

11. Verfahren nach einem der Ansprüche 1 bis 10 **dadurch gekennzeichnet, dass** nach der thermischen oxidativen Behandlung des Verwertungsschrittes mindestens ein Verwertungsprodukt ausgewählt aus CO₂, CO, H₂, H₂O erhalten wird.

12. Verfahren nach einem der Ansprüche 1 bis 10, **dadurch gekennzeichnet, dass** mindestens Kohlenmonoxid als Verwertungsprodukt erhalten wird und dieses als Reaktand dem Schritt A des Verfahrens zugeführt wird.

13. Produktionsanlage zur Herstellung organischer Isocyanate, mindestens umfassend eine Produktionseinheit (1) zur Herstellung von Phosgen aus Chlor und Kohlenmonoxid, die in gegebenenfalls absperrbarer Fluidverbindung (2) für den Phosgentransfer mit einer Produktionseinheit für organisches Isocyanat (3) steht, **dadurch gekennzeichnet, dass** ferner eine Verwertungseinheit (4) mit mindestens einer Heizquelle (5), mindestens einem Stoffzufluss (6) und mindestens einem Verwertungsproduktabfluss (7) vorhanden ist, wobei der Stoffzufluss (6) mit der Verwertungseinheit (4) und der Produktionseinheit (3) in gegebenenfalls absperrbarer Fluidverbindung steht, wodurch ein organischer Stoff aus einem Stoffstrom der Produktionsanlage in die Verwertungseinheit (4) eingebracht werden kann und darin durch Wärmeeintrag mittels Heizquelle (5) thermisch oxidativ behandelt werden kann und das resultierende Verwertungsprodukt durch den Verwertungsproduktabfluss (7) aus der Verwertungseinheit (4) gegebenenfalls nach Durchlauf mindestens einer Umsetzungsstufe (8) als Reaktand in die Produktionseinheit (1) oder in die Produktionseinheit für organisches Isocyanat (3) eingebracht werden kann.

14. Produktionsanlage nach Anspruch 13 **dadurch gekennzeichnet, dass** der Verwertungsproduktabfluss (7) mit der Produktionseinheit (1) in gegebenenfalls absperrbarer Fluidverbindung (9), insbesondere mit der Kohlenmonoxidzuführung (10) der Produktionseinheit (1), steht.

15. Produktionsanlage nach einem der Ansprüche 13 oder 14 **dadurch gekennzeichnet, dass** die Produktionseinheit für organisches Isocyanat (3) über den Stoffzufluss (6a) zunächst mit einer Isocyanat-Aufreinigungs-Einheit (11) in gegebenenfalls absperrbarer Fluidverbindung steht, wobei der Sumpf (12) der Isocyanat-Aufreinigungs-Einheit (11) über einen Abfluss (6b) für organischen Stoff des Stoffstroms in gegebenenfalls absperrbarer Fluidverbindung mit dem Stoffzufluss (6) der Verwertungseinheit (4) steht.
